(19) 

Europäisches
Patentamt

European
Patent Office

Office européen
des brevets

(11) **EP 4 327 743 A1**

(12) **EUROPEAN PATENT APPLICATION**
published in accordance with Art. 153(4) EPC

(43) Date of publication:
**28.02.2024   Bulletin 2024/09**

(21) Application number: **21947215.6**

(22) Date of filing: **25.06.2021**

(51) International Patent Classification (IPC):
**A61B 5/16** (2006.01)

(52) Cooperative Patent Classification (CPC):
**A61B 5/16**

(86) International application number:
**PCT/JP2021/025053**

(87) International publication number:
**WO 2022/269936 (29.12.2022 Gazette 2022/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Health Sensing Co., Ltd.**
**Hachioji-shi, Tokyo 192-0919 (JP)**

(72) Inventors:
- **KANEGAE, Masatomo**
  **Hachiouji-city, Tokyo 1920919 (JP)**
- **NIIZEKI, Kyuichi**
  **Hachiouji-city, Tokyo 1920919 (JP)**

(74) Representative: **Grünecker Patent- und
Rechtsanwälte
PartG mbB
Leopoldstraße 4
80802 München (DE)**

(54) **SLEEPING STATE ESTIMATION SYSTEM**

(57)     [Subject] To deliver a sleep state prediction system capable of predicting a sleep state including multiple sleep depths.

[Solution] A sleep state prediction system includes a phase coherence acquisition device that acquires phase coherence calculated based on a difference in instantaneous phase between a variation in heartbeat interval obtained from an animal during sleep and a breathing pattern; a body movement information acquisition device that acquires body movement information, a heartbeat information acquisition device that acquires heartbeat information from the animal simultaneously with the variation, or a respiratory information acquisition device that acquires respiratory information; and a sleep state prediction device that has a sleep state prediction model built-in, which is established through machine learning using sleep stages assessed by polysomnography on the animal as teaching data and at least 2 sets of simultaneously acquired data on phase coherence, body movement, heartbeat, and respiration as input data. The system inputs at least 2 data sets in the sleep state prediction model to predict a sleep state of the animal.

FIG.1

**Description**

Technical Field

The embodiments are related to a sleep state prediction system that predicts a sleep state based on biological information acquired from an animal, including the sleep state prediction system that predicts a sleep state according to phase coherence calculated based on a difference in instantaneous phase between a variation in heartbeat interval acquired during sleep of the animal and a breathing pattern of the same animal simultaneously acquired with the variation in heartbeat interval.

Background Art

It is said that human sleep is largely divided into non-rapid eye movement (non-REM) sleep and REM sleep, which are alternated at an interval of approximately 90 minutes. To assess quality and quantity of sleep (sleep cycle and sleep depth) objectively, polysomnograph is necessary. The polysomnography is performed at a medical institution or with the apparatus rented from the medical institution and involves the monitoring of sleeping subjects through the continuous and simultaneous recording of brain waves, electrocardiogram, electromyogram, and electrooculogram, accompanying complicated handling to identify sleep stages.

In addition, the multiple sensors have to be attached to various sites of the body, restricting movements of a subject. Unconscious movements during sleep may take the sensors off, resulting in failures of signal detection and state monitoring. Thus, such measurement has problems.

Furthermore, the polysomnographic apparatus is large and utilizes multiple physiological parameters that cannot be assessed by an individual. Current measurement situations have not allowed individuals to assess the sleep quality at home. Therefore, an alternative biometric method comparable to the polysomnography that allows individuals to monitor the physical state and sleep quality readily have been demanded.

PTL1 discloses that a sleep state measuring device including a phase coherence calculation device that calculates phase coherence based on a difference in instantaneous phase between a variation in heartbeat interval acquired during sleep of an animal and a corresponding simultaneous breathing pattern of the animal. The sleep state measuring device determines a sleep state by measuring the phase coherence, which correlates to $\delta$ waves in an electroencephalogram during sleep.

PTL1 also discloses a method to calculate phase coherence as follows: bio-vibration signals are detected with a sheet-type piezoelectric sensor and used to obtain heartbeat intervals and breathing patterns; respiratory sinus arrhythmia and the breathing patterns are subjected to Hilbert transformation to obtain analytic signals, from which instantaneous phases are determined; and a phase difference between the determined instantaneous phases is used to calculate the phase coherence.

Citation List

Patent Literature

PTL1: Japanese Patent No. 6358768

Summary of Invention

Technical Problem

Sleep is largely classified into REM sleep or non-REM sleep, and the non-REM sleep is further divided into 4 stages ranging from Stage I to Stage IV. Stage I (relaxed wakefulness, beginning of sleep) is a dozing state, in which $\alpha$ waves are losing rhythm and gradually becoming flat. Stage II is characterized by a deeper sleep state than that at Stage I, in which sleep spindles (SPINDLE) and K-complexes appear. Stage III is characterized by a substantially deep sleep state, in which $\delta$ waves account for not less than 20% and less than 50% of brain waves. Stage IV is characterized by a deepest sleep state, in which $\delta$ waves account for not less than 50%. In Stages III and IV of non-REM sleep, slow waves constitute a large percentage of brain waves, and thus sleep at these stages is also called slow wave sleep. The American Academy of Sleep Medicine classifies Stage I as N1, Stage II as N2, and Stages III and IV collectively as N3.

REM sleep, on the other hand, is characterized by brain waves in a low amplitude fast wave pattern similar to those of an awake subject but by a deep sleep state that requires stronger stimulation for awakening than the slow wave sleep state and associated with rapid eye movement. In a healthy adult, these two sleep states are alternated at an interval of approximately 90 minutes (sleep cycle), forming one-night sleep. Sleep starts in a form of non-REM sleep at Stage I and deepens entering Stage II and reaching Stages III and IV, then shallows transiently, and finally is transformed into the REM sleep. A latter half of the sleep repeatedly alternates between shallow non-REM sleep and REM sleep and finally ends with awakening. As described above, in an electroencephalogram, $\delta$ waves at frequency of 1 to 4 Hz appear during slow wave sleep, and thus $\delta$ waves can be used as an indicator of sleep depth.

The sleep state measuring device disclosed in PTL1 assesses a state with calculated phase coherence greater than a threshold as deep sleep and one with calculated phase coherence smaller than the threshold as shallow sleep and evaluates sleep quality based on duration in which phase coherence remains greater than the threshold and cycle of fluctuations of the phase coherence.

In addition, PTL1 discloses that the threshold may be set at multiple levels for step-wise evaluation of the sleep quality.

The sleep state, however, is very complicated as described above, and thus there is a demand for a sleep state prediction system that can predict the sleep state as with polysomnography, which indicates sleep depth.

The embodiments are intended to solve a part of the above problem and thus to provide a sleep state prediction system that can predict a sleep state including multiple sleep depths as with conventional polysomnography, which indicates a sleep state.

Solution to Problem

To solve the above problem, the sleep state prediction system of the embodiments predicts a sleep state of an animal from calculation results presented by a sleep state prediction model, the system comprising:

A phase coherence acquisition device that acquires phase coherence calculated based on a difference in instantaneous phase between a variation in heartbeat interval obtained from the animal during sleep and a breathing pattern of the animal simultaneously obtained with the variation in heartbeat interval;

A body movement information acquisition device that acquires body movement information from the animal simultaneously with the variation in heartbeat interval a heartbeat information acquisition device that acquires heartbeat information from the animal simultaneously with the variation in heartbeat interval, or a respiratory information acquisition device that acquires respiratory information from the animal simultaneously with the variation in heartbeat interval; and

A sleep state prediction device that has a sleep state prediction model built-in, which is established through machine learning using sleep stages assessed by polysomnography on the animal as teaching data and at least 2 sets of data on 4 parameters of phase coherence, body movement, heartbeat, and respiration determined from bio-vibration signals acquired simultaneously during the polysomnography as input data.

The sleep state prediction device inputs in the sleep state prediction model the same type of data as that of the input data, which are chosen from data on the following parameters, and the said sleep state prediction model presents calculation results for the prediction: phase coherence obtained from the phase coherence acquisition device in which phase coherence calculated from the variation in heartbeat interval obtained from heartbeat information acquisition device and breathing pattern obtained from the respiratory information acquisition device; body movement obtained from the body movement information acquisition device; heartbeat obtained from the heartbeat information acquisition device; and respiration obtained from the respiratory information acquisition device.

In the sleep state prediction system, the phase coherence acquisition device may also calculate phase coherence based on a difference in instantaneous phase between a variation in heartbeat interval acquired with the heartbeat information acquisition device and a breathing pattern acquired with the respiratory information acquisition device.

Furthermore, the variation in heartbeat interval and the heart rate acquired with the heartbeat information acquisition device may be calculated from predicted ECG signals based on bio-vibration signals, which have been used as input data, determined by integration of a power of the frequency component ranging from 5 Hz to 15 Hz, which is a part of the frequency components derived from bio-vibration signals by continuous wavelet transform (CWT), or calculated from signals obtained by passing bio-vibration signals through a band-pass filter (BPF) with a pass-band of 5 Hz to 15 Hz.

A breathing pattern and respiratory rate acquired with the respiratory information acquisition device may be also calculated from signals obtained by passing bio-vibration signals through a low-pass filter with the upper frequency of 0.5 Hz.

In addition, body movements information may be the number of body movements (the number of peaks exceeding a predetermined threshold among bio-vibration signals), the number of body movements in the predetermined period of time, or the integration value of body movement signals in the predetermined period of time.

Advantageous Effects of Invention

According to the embodiments, one non-restraint sensor such as a vibration sensor is able to provide a result on sleep stage equivalent to that assessed by polysomnography as well as provide detailed data related to sleep, more specifically, information on times of beginning and end of sleep, duration and proportion of deep sleep, REM sleep duration, wake time, sleep efficiency, and sleep cycle.

[Brief Description of Drawings]

[FIG. 1] is a block diagram of the sleep state prediction system.
[FIG. 2(A)] and [FIG. 2(B)] are conceptual diagrams of the sleep state prediction mode and learning mode.
[FIG. 3(A)] and [FIG.3(B)] are flow charts of sleep state prediction processing.
[FIG. 4] illustrates heartbeat information obtained by band-pass filtering.
[FIG. 5] illustrates heartbeat information obtained by CWT.
[FIG. 6] illustrates heartbeat information obtained from predicted ECG signals.
[FIG. 7] illustrates 10-second moving averages of heart rate (HR), respiratory rate (RR), phase coherence (λ), and body movement (BM) obtained from bio-vibration signals acquired with the vibration sensor.
[FIG. 8] illustrates sleep state as well as 30-second interval signal wave forms of the 10-second moving averages of heart rate (HR), respiratory rate (RR), phase coherence (λ), and body movements (BM).
[FIG. 9] illustrates ECG-derived signal wave forms of the heart rate (HR) and phase coherence (λ) in FIG. 8.
[FIG. 10(A)] illustrates sleep stages assessed by polysomnography (measured values); [FIG. 10(B)] illustrates predicted sleep states; and
[FIG. 10(C)] illustrates a confusion matrix between prediction results and true values (measured values).
[FIG. 11(A)] illustrates sleep stages assessed by polysomnography (measured values); [FIG. 11(B)] illustrates predicted sleep states; and
[FIG. 11(C)] illustrates a confusion matrix between prediction results and true values (measured values).
[FIG. 12(A)] illustrates sleep stages assessed by polysomnography (measured values); [FIG. 12(B)] illustrates predicted sleep states; and
[FIG. 12(C)] illustrates a confusion matrix between prediction results and true values (measured values).
[FIG. 13(A)] illustrates sleep stages assessed by polysomnography (measured values); [FIG. 13(B)] illustrates predicted sleep states; and
[FIG. 13(C)] illustrates a confusion matrix between prediction results and true values (measured values).
[FIG. 14(A)] illustrates sleep stages assessed by polysomnography (measured values); [FIG. 14(B)] illustrates predicted sleep states; and
[FIG. 14(C)] illustrates a confusion matrix between prediction results and true values (measured values).

Description of Embodiments

Sleep State Prediction System

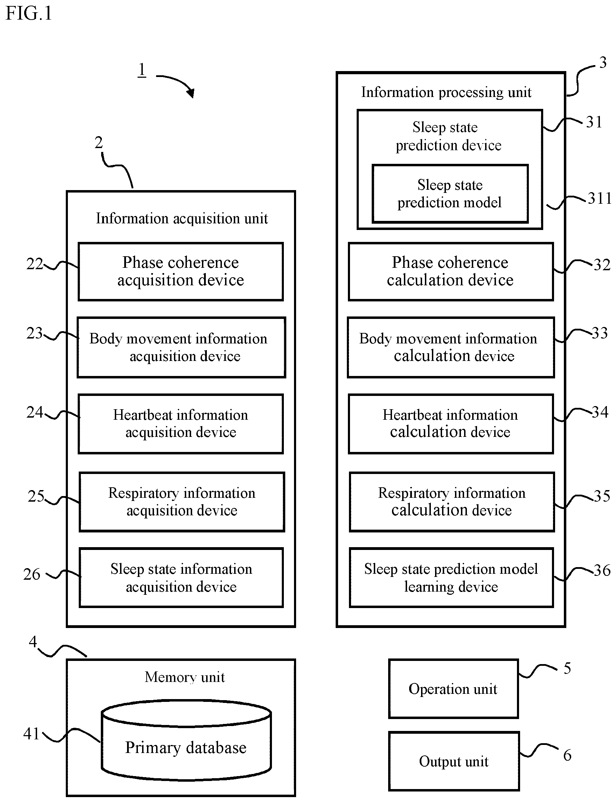

As illustrated in FIG. 1, a sleep state prediction system of the embodiments is at least equipped with an information acquisition unit 2 and an information processing unit 3. The sleep state prediction system 1, furthermore, may be equipped with a memory unit 4, an operation unit 5, and an output unit 6.

An information acquisition unit 2 includes one or more of a phase coherence acquisition device 22, a body movement information acquisition device 23, a heartbeat information acquisition device 24, and a respiratory information acquisition device 25 and may include other device to acquire further information (image, voice, temperature, and other data).

An information processing unit 3 includes a sleep state prediction device 31, may include further one or more of a phase coherence calculation device 32, a body movement information calculation device 33, a heartbeat information calculation device 34, and respiratory information calculation device 35, and furthermore may include a sleep state prediction model learning device 36.

The sleep state prediction system 1 may be implemented with one apparatus or with multiple units or parts connected through a network or other modalities.

The sleep state prediction system 1 of the embodiments, for example, may be implemented with a sensor, personal digital assistants (PDAs) (mobile phone, smartphone, and other devices) connected to the sensor, and a sever connected to the PDAs through a network. In this system, the sensor functions as the information acquisition unit 2; the PDAs function as various information calculation devices and a phase coherence calculation device, which are a part of the information processing unit 3; and the server functions as a sleep state prediction device 31.

In addition, a sleep state information acquisition device 26 and a sleep state prediction model learning device 36 are not essential components of the sleep state prediction system 1 but are used to operate the sleep state measuring system 1 as a sleep state prediction model learning system.

The information acquisition unit 2 acquires information necessary for sleep state prediction and thus may include a sensor for measurement on animals and an input unit that inputs information from the sensor in a wired or wireless mode or include an input unit that can input already measured or calculated information from a recording medium.

The information acquisition unit 2, in other words, is at least equipped with the input unit that input information and may be equipped with the sensor for measuring biological information connected to the input unit in a wired or wireless mode where necessary. Depending on information acquired, the information acquisition unit 2 functions as the phase coherence acquisition device 22, the body movement information acquisition device 23, the heartbeat information acquisition device 24, the respiratory information acquisition device 25, a sleep state information acquisition device 26, or the other devices.

The information processing unit 3 processes input information and, for example, can utilize a processing function of a central processing unit (CPU) in a computer. A part of information processing can be also implemented with an analog circuit but not a digital circuit.

Frequency filter processing, for example, may be implemented with an analog filter such as a low-pass filter (LPF) and a high-pass filter (HPF) including condenser, resistance, operational amplifier, and other devices or may be implemented with a digital filter that performs filtering with a processing function of the CPU.

The information processing unit 3 may include both digital and analog circuits according to a type of information processing, and input information in an analog format, where applicable, may be converted into digital signals through an analog-digital conversion circuit.

The information processing unit 3 is required to perform a different function or processing depending on the input information, but it functions as a sleep state prediction device 31, a phase coherence calculation device 32, a body movement information calculation device 33, a heartbeat information calculation device 34, a respiratory information calculation device 35, a sleep state prediction model learning device 36, or other devices according to information to be processed.

The phase coherence acquisition device 22 may be an input unit that can input already calculated phase coherence in a wired or wireless mode or may acquire calculated phase coherence from the phase coherence calculation device 32, which receives necessary information from the information acquisition unit 2.

The phase coherence calculation device 32 calculates phase coherence from various information. To calculate phase coherence, for example, the following information may be input.

A) A difference in instantaneous phase between a variation in heartbeat interval and a breathing pattern in the same time series is input in the information acquisition unit 2, and then phase coherence is calculated using the instantaneous phase difference input by the phase coherence calculation device 32 in the information processing unit 3.
B) An instantaneous phase of a variation in heartbeat interval and that of a breathing pattern in the same time series are input in the heartbeat information acquisition device 24 and the respiratory information acquisition device 25 in the information acquisition unit 2, and a difference between the above phases is calculated by an instantaneous phase difference calculation function of the information processing unit 3. Using the calculated instantaneous phase differences, phase coherence is calculated by the phase coherence calculation device 32.
C) A variation in heartbeat interval and a breathing pattern in the same time series (including a detection result with a sensor) are input in the heartbeat information acquisition device 24 and the respiratory information acquisition device 25 in the information acquisition unit 2. The heartbeat information calculation device 34 and the respiratory information calculation device 35 in the information processing unit 3 calculate an instantaneous phase of the variation in heartbeat interval and that of the breathing pattern. Using the calculated instantaneous phases, phase coherence is calculated by the instantaneous phase difference calculation function and the phase coherence calculation device 32.
D) Biological information including that on heartbeat and biological information including that on respiration (including the detection result with the sensor) are input in the heartbeat information acquisition device 24 and the respiratory information acquisition device 25, respectively, in the information acquisition unit 2. The heartbeat information calculation device 34 and the respiratory information calculation device 35, which have a heartbeat interval calculation function and a breathing pattern calculation function, respectively, in the information processing unit 3 calculate the variation in heartbeat interval and the breathing pattern from the former biological information and the breathing pattern from the latter biological information through their functions. Then, the same processing as that in the above C) is performed.
The biological information including that on heartbeat may include electrocardiogram (ECG) separately predicted from bio-vibration information or be ECG separately acquired.

E) Biological information including both information on heartbeat and that on respiration (including the detection result with the sensor) is input in the information acquisition unit 2. From the above biological information, the heartbeat information calculation device 34 and the respiratory information calculation device 35 in the information processing unit 3 calculate information on heartbeat and that on breathing pattern. Information on the detected or calculated heartbeat or breathing pattern may be used in the subsequent processing.

The phase coherence is calculated from a difference between the instantaneous phase $\psi h(t)$ of the variation in heartbeat interval associated with breathing and the instantaneous phase $\psi r(t)$ of the breathing pattern, both of which can be calculated. The instantaneous phase $\psi h(t)$ of the variation in heartbeat interval can be calculated as follows: a temporal change ($S(t)$) in the variation in heartbeat interval associated with breathing is calculated from data on heartbeat interval and then turned into an analytic signal by Hilbert transformation provided in the formula below (1). H [...] in the formulae (1) and (2) is a Hilbert transformation operator, and P.V. stands for the Cauchy's principal value.

$$\mathrm{H}[S(t)] = \frac{1}{\pi} P.V. \int_{-\infty}^{\infty} \frac{S(\tau)}{t-\tau} d\tau, \quad \psi_h(t) = tan^{-1} \frac{H[S(t)]}{S(t)} \qquad (1)$$

The instantaneous phase $\psi h(t)$ of the breathing pattern can be calculated as follows: a temporal change ($R(t)$) in the breathing pattern is calculated from information on breathing pattern and then turned by Hilbert transformation provided in the formula below (2).

$$\mathrm{H}[R(t)] = \frac{1}{\pi} P.V. \int_{-\infty}^{\infty} \frac{R(\tau)}{t-\tau} d\tau, \quad \psi_r(t) = tan^{-1} \frac{H[R(t)]}{R(t)} \qquad (2)$$

The instantaneous phase difference $\Psi(t)$ can be calculated according to the formula (3) below using the instantaneous phase $\psi h(t)$ of the variation in heartbeat interval and that $\psi r(t)$ of the breathing pattern, which have been provided by the formulae (1) and (2).

$$\Psi(t) = \psi h(t) - \psi r(t) + 2n\pi \qquad (3)$$

Where, n is an appropriate integer that leads to $-\pi \leq \Psi \leq \pi$.

Then, phase coherence at a time point ($t_k$) can be determined by averaging N data sets of the instantaneous phase difference between the time points $t_k - N/2$ and $t_k + N/2$ according to the formula (4), where N is a number of data sets in the calculation window.

$$\lambda(t_k) = \left| \frac{1}{N} \sum_{j=k-N/2}^{k+N/2} e^{i\Psi(t_j)} \right| \qquad (4)$$

The phase coherence takes values between 0 and 1. As the instantaneous phase difference between the variation in heart rate interval and breathing pattern is reaching a constant relationship, the phase coherence value is becoming approximate to 1. As the instantaneous phase difference is reaching a random state, it is becoming approximate to 0.

As an amplitude of a $\delta$ wave in an electroencephalogram is increased, the phase coherence ($\lambda$) tends to become approximate to 1. As the amplitude of a $\delta$ wave is decreased, it tends to become approximate to 0.

When a subject is relaxed at rest, the phase coherence is approximate to 1. When a subject is under mental stress, it is reduced. The phase coherence value therefore can be used as a normalized indicator for prediction of mental stress.

The body movement information acquisition device 23 may be an input unit that can receive already calculated body movement information in a wired or wireless mode or acquire body movement information calculated from detection results with various sensors by the body movement information calculation device 33 in the information processing unit 3.

The body movement information calculation device 33 calculates body movement information from various information by choosing processing appropriate for a type of the sensor or input signals.

The body movement information is a component available for machine learning and may include a number of body movements, a frequency of body movements in a predetermined period of time, an integral of body movement signals in a predetermined period of time, and other values on body movements.

The predetermined period of time is a unit used to divide data, preferably matches unit of time used in the other input data, and may be specified, for example, as 10, 20, 30, 40, or 50 seconds or 1 minute.

A sensor for acquiring the body movement information can be, for example, a vibration sensor such as a piezoelectric sensor, an accelerometry sensor, an electromagnetic body movement sensor capable of detecting body movements with electromagnetic waves, or an electromyograph.

Because bio-vibration signals detected with the vibration sensor include ballistocardiac movements derived from heartbeats, vibrations derived from respiratory chest movements, and vibrations derived from body movements, vocalization, and an external environment, the body movement information calculation device 33 separates and extracts body movement-derived vibration signals from the bio-vibration signals, for example, by passing the signals through a high-pass filter (HPF) or a band-pass filter (BPF) with the lower limit of frequency of 10 Hz or higher.

The body movement-derived vibration signals or body movement information may be calculated as the number of body movements by counting the number of peaks exceeding a predetermined threshold among calculated body movement-derived vibration signals or as the frequency of body movements in the predetermined period of time by specifying the predetermined period of time and counting the number of body movements in this period of time. Or the integration value of body movement signals in the predetermined period of time may be calculated.

The body movement information acquisition device 24 may be an input unit that can receive already calculated body movement information in a wired or wireless mode or acquire body movement information calculated from detection results with various sensors by the heartbeat information calculation device 34 in the information processing unit 3.

The heartbeat information calculation device 34 calculates heartbeat information from various measures of heart rate variability by choosing processing appropriate for a type of the sensor or input signals.

The heartbeat information is a component usable for machine learning, and may include, for example, heart rate (HR), coefficient of variation of heart rate ($CV_{HR}$), deviation of heart rate ($D_{HR}$), coefficient of variation of deviation of heart rate ($CV_{DHR}$), standard deviation of heart rate ($SD_{HR}$), coefficient of variation of standard deviation of heart rate ($CV_{SDHR}$), heartbeat interval (RRI), coefficient of variation of heartbeat interval ($CV_{RRI}$), deviation of heartbeat interval ($D_{RRI}$), coefficient of variation of deviation of heartbeat interval ($CV_{DRRI}$), standard deviation of heartbeat interval ($SD_{RRI}$), coefficient of variation of standard deviation of heartbeat interval ($CV_{SDRRI}$), variation of heartbeat interval ($V_{RRI}$), coefficient of variation of variation of heartbeat interval ($CV_{VRRI}$), instantaneous amplitude of variation of heartbeat interval (A(t)), respiratory sinus arrhythmia (RSA), and coefficient of variation of respiratory sinus arrhythmia ($CV_{RSA}$). Data on any heartbeat parameter are preferably normalized with a mean value of the whole data acquired during sleep. For example, in a sleep state prediction model 311, normalized heart rates obtained by calculating ratios of acquired heart rates with respect to the mean value of the whole data (each heart rate/mean value) may be input as the heartbeat information.

Normalization of the heartbeat information and respiratory information reduces individual differences of subjects and thereby increases versatility and measurement precision of the sleep state prediction model 311. Because normalization is performed on the mean value obtained after acquisition of the whole data during sleep, sleep state cannot be predicted on a real-time basis. The real-time sleep state may be calculated using raw data (heart rate, respiratory rate, or other parameters) or late normalized data.

A sensor for acquiring the heartbeat information can be, for example, a sensor for measurement of ECG, a pulse wave sensor, or a vibration sensor. The heartbeat information calculation device 34 can calculate various heartbeat information by removing noise (body movement component and other components) from ECG wave forms detected with the sensor for measurement of ECG or pulse waves detected with the pulse wave sensor where necessary.

Because the bio-vibration signals detected with the vibration sensor include ballistocardiac movements derived from heartbeats, vibrations derived from respiratory chest movements, and vibrations derived from body movements, vocalization, and an external environment, the heartbeat information calculation device 34 separates and extracts the heartbeat-derived ballistocardiac movements from the bio-vibration signals, for example, by passing the signals through the band-pass filter (BPF) with a pass-band of 0.5 Hz to 10 Hz. Furthermore, various heartbeat information can be calculated from the separated and extracted ballistocardiac movements.

The heart rate can be also obtained as follows: to extract a heartbeat component, bio-vibration signals obtained with the vibration sensor are passed through a band-pass filter (BPF) with a pass-band of 5 Hz to 15 Hz for absolutization followed by smoothing with a time constant of 0.2 seconds and passing through a high-pass filter with the cutoff frequency of 0.3 Hz, and the subsequently obtained signals are used to derive predicted ECG signals, in which peaks are then counted to obtain the heart rate. A function to predict ECG (no figures illustrated) may be included in the information processing unit 3 or in other calculation tools connected to the said sleep state prediction system in a wired or wireless mode.

In addition, the heart rate can be obtained by counting peaks in directly obtained ECG signals

The heart rate may be also obtained as follows: bio-vibration signals obtained with the vibration sensor are subjected to continuous wavelet transform (CWT) to derive frequency components, and a power of the frequency component ranging from 5 Hz to 15 Hz is integrated to obtain signals, in which peaks are then counted to obtain the heart rate.

The respiratory information acquisition device 25 may be an input unit that can receive already calculated respiratory information in a wired or wireless mode or acquire respiratory information calculated from detection results with various sensors by the respiratory information calculation device 35 in the information processing unit 3. The respiratory information calculation device 35 calculates respiratory information from various measures of respiration by choosing processing appropriate for a type of the sensor or input signals.

The respiratory information is a component available for machine learning, and may include, for example, respiratory rate (RR), coefficient of variation of respiratory rate ($CV_{RR}$), deviation of respiratory rate ($D_{RR}$), coefficient of variation of deviation of respiratory rate ($CV_{DRR}$), standard deviation of respiratory rate ($SD_{RR}$), coefficient of variation of standard deviation of respiratory rate ($CV_{SDRR}$), breathing interval (BI), coefficient of variation of breathing interval ($CV_{BI}$), deviation of breathing interval ($D_{BI}$), coefficient of variation of deviation of breathing interval ($CV_{DBI}$), standard deviation of breathing interval ($SD_{BI}$), coefficient of variation of standard deviation of breathing interval ($CV_{SDBi}$), number of snoring per unit of time, and instantaneous amplitude of breathing pattern (A(t)).

Data on any respiratory parameter are preferably normalized with a mean value of the whole data acquired during sleep. For example, in a sleep state prediction model 311, normalized respiratory rates obtained by calculating ratios of acquired respiratory rates with respect to the mean value of the whole data (each respiratory rate/mean value) may be input as the respiratory information.

A sensor for acquiring the respiratory information can be, for example, a respiration sensor, a sensor for measurement of ECG, a pulse wave sensor, a vibration sensor such as a piezoelectric sensor, or an electromagnetic respiration sensor capable of detecting thoracic movements with electromagnetic waves. The respiratory information calculation device 35 can calculate various respiratory information by removing noise (signals derived from snoring, sleep talking, and other activities during sleep) from signals representative of temporal changes in breathing pattern detected with the respiration sensor where necessary.

The respiratory information calculation device 35 can separate and extract a respiratory component from ECG wave forms detected with the sensor for measurement of ECG, pulse waves detected with the pulse wave sensor, and bio-vibration signals detected with the vibration sensor, for example, by passing such data through a low-pass filter (LPF) with the upper frequency of 1 Hz or lower or the band-pass filter (BPF) and furthermore, from the separated and extracted respiratory component, calculate various respiratory information.

The vibration sensor, if included in the information acquisition unit 2, may operate in either contact or non-contact mode.

The sensor measuring vibrations in a contact mode can detect ballistocardiogram wave forms or bio-vibration signals when placed in contact with an animal directly or indirectly. The sensor measuring vibrations in a contact mode to detect ballistocardiogram wave forms or bio-vibration signals is placed on or in proximity to various vibration-generating animals, detect vibrations from the animals, and output these as electronic signals.

For the sensor measuring vibrations, a piezoelectric element is desirably used as a piezoelectric sensor, but the other sensors such as a polymer piezoelectric element (polyolefin material) may be used. A material for the piezoelectric element may be, for example, porous polypropylene electret film (electro mechanical film, EMFI), polyvinylidene difluoride film (PVDF), poly[(vinylidenefluoride-co-trifluoroethylene] (P(VDF-TrFE)), or poly[(vinylidenefluoride-co-tetrafluoroethylene] (P(VDF-TFE)). The piezoelectric sensor is desirably in a film form.

Furthermore, the piezoelectric sensor is desirable, because it is capable of acquiring ballistocardiogram wave forms or bio-vibration signals without restraining the animal, allowing measurement in a more stress-free state. The piezoelectric sensor, however, may be used as a wearable sensor attached to a wristband, belt, watch, ring, or headband worn by the animal.

In addition, the other types of sensors measuring vibrations may be used to acquire ballistocardiogram wave forms or bio-vibration signals: for example, a highly sensitive accelerometry sensor may be attached in contact with the body like a watch or portable terminal or integrated in a part of a bed or chair; or a pressure sensor that detects changes in air or liquid pressure in a tube may be used.

Furthermore, for measuring vibrations, a non-contact sensor that can acquire ballistocardiogram wave forms or bio-vibration signals in a non-contact mode through signal transmission using microwaves or other waves may be used. Ballistocardiogram wave forms or bio-vibration signals acquired from the following data may be used: data obtained with a microwave Doppler sensor; data obtained with receiving waves that facilitate determination of a distance from an object based on reflection delay time of ultrawide band (UWB) impulses; data obtained with electromagnetic waves other than microwaves; data obtained with reflected or transmitted beam using LED light; and data obtained with reflected waves of ultrasonic waves. These sensors using microwaves and other waves can be downsized and capable of acquiring signals in a non-contact and non-restraint manner under a remote operating condition. In addition, the accelerometry sensor can be downsized as well.

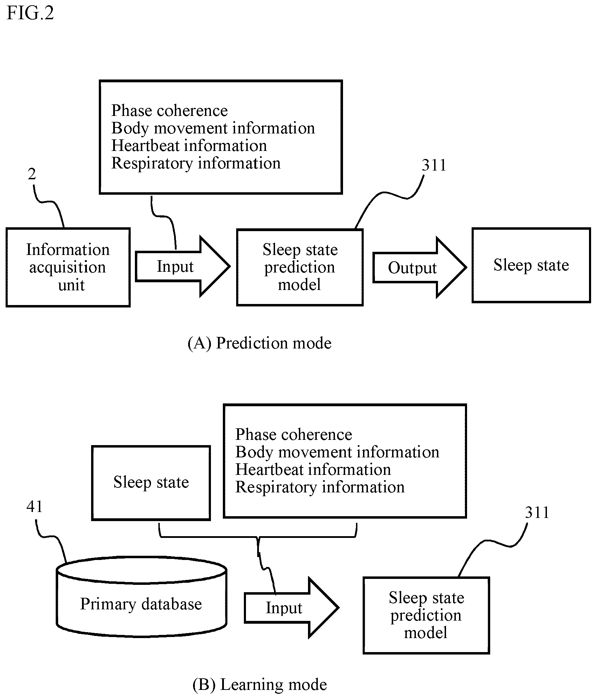

As illustrated in FIG. 2(A), the sleep state prediction device 31 inputs information from the information acquisition unit 2 in the sleep state prediction model 311, which is built in the device and, upon receiving the information, performs calculation to predict a sleep state of the animal. The sleep state prediction device 31 then receives the sleep state as a prediction result from the sleep state prediction model 311.

The sleep state is output as the prediction result through the output unit 6 where necessary. The information input in the sleep state prediction model 311 is used to establish the model and includes at least one data set chosen from phase coherence acquired with the phase coherence acquisition device, body movements information acquired with the body movement information acquisition device, heartbeat information acquired with the heartbeat information acquisition device, and respiratory information acquired with the respiratory information acquisition device. More specifically, phase coherence and body movement information are preferably used as input data for establishing the sleep state prediction model 311. Furthermore, in addition to the phase coherence and the body movement information, at least 2 data sets of the heartbeat information and at least 2 data sets of the respiratory information may be used as input data for establishing the model.

For example, when 4 data sets on phase coherence ($\lambda$), body movement frequency per unit of time (30 seconds), heart rate (HR), and respiratory rate (RR) are used to establish the sleep state prediction model 311, the sleep state prediction device 31 inputs in the sleep state prediction model 311 the 4 data sets acquired with the information acquisition unit 2 and calculated with the information processing unit 3 and then receives the sleep state as output from the model.

The sleep state prediction model 311 comprises information (program or data structure) that receives multiple data sets including data on phase coherence and predicts the sleep state of the animal during data acquisition (awake, REM sleep, or non-REM sleep state or its stage [N1, N2, and N3]) by calculation on these data sets. In addition, as illustrated in FIG. 2 (B), the sleep state prediction model learning device 36 executes machine learning based on a primary database 41 including the sleep state and corresponding phase coherence as well as additional input data and thus is used to establish the sleep state prediction model 311.

In other words, from big data accumulated in the primary database 41 including the sleep state and corresponding phase coherence as well as additional input data, necessary data are input in the sleep state prediction model 311 for learning. For example, the sleep state prediction model learning device 36 inputs the following data in the sleep state prediction model 311: data on the sleep state acquired with the sleep state information acquisition device 26 and phase coherence calculated from data acquired in the same time series as that for the sleep state (or simultaneous data); and at least one simultaneous data set of the body movement information acquired with the body movement information acquisition device, the heartbeat information acquired with the heartbeat information acquisition device, and the respiratory information acquired with the respiratory information acquisition device.

Methods available for machine learning include Naive Bayes classifier, Neural Network, Deep Learning, decision tree, Support Vector Machine, discriminant analysis, and nearest neighbor algorithm. Of these, one method may be used alone or an ensemble learning method using 2 or more methods in combination may apply as well. Examples of Neural Network used for machine learning includes Convolutional Neural Network (CNN), Recurrent Neural Network (RNN), and Long Short-Term Memory (LSTM) Neural Network (Recurrent Neural Network capable of learning long-term dependence). To capture a transition trend of the sleep state, neural networks capable of learning recurrent linkages such as RNN and LSTM are desirable.

The memory unit 4 can store information acquired with the information acquisition unit 2, calculation results presented by the information processing unit 3, assessment results presented by a assessment function, and other types of information and has the primary database 41.

The primary database 41 stores data on the sleep state acquired from the animal during sleep and phase coherence calculated from data acquired in the same time series as that for the sleep state (or simultaneous data) as well as simultaneous data sets of body movement information, heartbeat information and respiratory information as big data.

Furthermore, the primary database 41 may include information on physical characteristics such as species, sex, age, height, and body weight of the animal subject to measurement, information on time such as date of measurement, sleep time, time of bedding, time of rising, and season, and information on environment such as temperature and humidity, weather, and noise in a measurement place.

Data on individual sleep states and corresponding phase coherence as well as various data sets of body movement information, heartbeat information and respiratory information stored in the primary database 41 are used in learning of the sleep state prediction model 311.

Furthermore, information on physical characteristics, time, and environment may be input in the sleep state prediction model 311 as characteristic variables.

In addition, multiple sleep state prediction models 311 may be established according to the information on physical characteristics, time, and environment. For example, the sleep state prediction model in male adults by age category (aged ≥10 and <20, ≥20 and <30, and ≥30 and <60 years), that in people who have taken daytime sleep, and that during a torrid night with the lowest temperature ≥25°C are established, and from these established models, the sleep state prediction model to be used for predicting the sleep state may be chosen based on a situation of the subject.

The operation unit 5 is provided with operation terminals such as a switch, a touch panel, a button, a knob, a keyboard, a mouse, and a voice input microphone for a user to operate a sleep state measurement device 1. In addition, the operation unit 5 may be provided with a display indicating details of operations.

The output unit 6 may output a predicted sleep state and various acquired information. The output unit 6 may use a display to present a result in an image, a printer to output the result in a paper form, a speaker to output the result in a voice, and a wired or wireless output terminal to output the result as electronic information.

The display of the output unit 6 may be substituted by the touch panel or the display indicating details of operations in the operation unit 5.

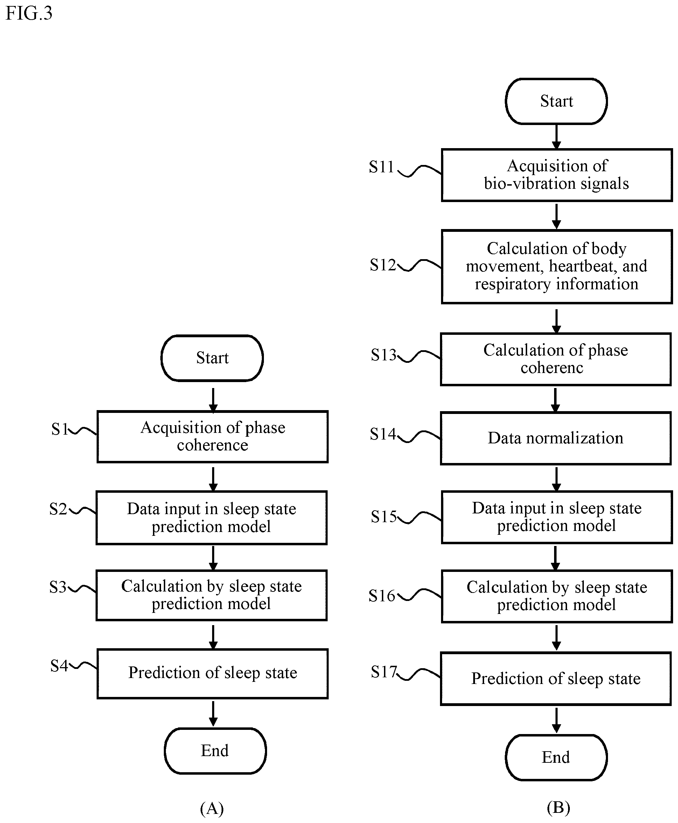

FIG. 3(A) illustrates a flow chart of sleep state prediction processing in an implemented form of the sleep state prediction system 1. At S1, the sleep state prediction system 1 acquires phase coherence and corresponding simultaneous data to input in the sleep state prediction model 311 through the information acquisition unit 2. At S2, the sleep state prediction device 31 inputs phase coherence and the acquired data in the sleep state prediction model 311. At S3, the sleep state prediction model 311 calculates the most probable sleep state of the animal from the input phase coherence and data. At S4, after the calculation, the sleep state prediction model 311 predicts and outputs the most likely sleep state of the animal during measurement of input phase coherence and data.

FIG. 3(B) illustrates a flow chart of sleep state prediction processing in another implemented form of the sleep state prediction system 1.

At S11, the sleep state prediction system 1 acquires bio-vibration signals detected with the vibration sensor.

At S12, the information processing unit 3 calculates various information from the acquired bio-vibration signals. The body movement information calculation device 33 calculates body movement information from the bio-vibration signals. The heartbeat information calculation device 34 calculates at least the heart rate and the instantaneous phase of a variation in heartbeat interval as the heartbeat information from the bio-vibration signals. The respiratory information calculation device 35 calculates at least the respiratory rate and the instantaneous phase of a breathing pattern as the respiratory information from the bio-vibration signals.

At S13, the phase coherence calculation device 32 calculates phase coherence of the instantaneous phase difference between a variation in heartbeat interval and a breathing pattern, which have been calculated by the heartbeat information calculation device 34 and the respiratory information calculation device 35, respectively.

At S14, the calculated heartbeat information and respiratory information are normalized with a mean of the whole data during sleep. The information processing unit 2 executes normalization. More specifically, the unit calculates the mean from the whole data during sleep stored in the primary database 41 and normalizes the heartbeat information and the respiratory information with the calculated mean.

Then, at S15, the sleep state prediction device 31 inputs phase coherence and at least 2 data sets of the normalized heartbeat information, respiratory information, and body movements information in the sleep state prediction model 311.

At S16, the sleep state prediction model 311 calculates the most likely sleep state of the animal from the input phase coherence and at least 2 data sets of the normalized heartbeat information, respiratory information, and body movements information.

At S17, after the calculation, the sleep state prediction model 311 predicts and outputs the most likely sleep state of the animal during measurement of input phase coherence as well as the heartbeat information, the respiratory information, and the body movements information.

EXAMPLE 1

In this EXAMPLE, the sleep state was predicted using the sleep state prediction model 311 in which learning had been performed according to Bidirectional Long Short-Term Memory (BiLSTM) neural network.

The BiLSTM neural network is a model capable of leaning directional long-term dependence by linking short-term memory with long-term memory. BiLSTM in this EXAMPLE has a structure including an input layer of sequence input, a hidden layer of 3 BiLSTM layers, and an output layer of 6-step classification output (leaving bed [LV], awake [WK], REM, N1, N2, and N3). Each BiLSTM layer includes 128 hidden units.

In a verification study, bio-vibration signals were acquired from 8 subjects simultaneously during polysomnography, and from the said signals, phase coherence ($\lambda$), body movement information (BM), heart rate (HR), and respiratory rate (RR) were obtained and subjected to machine learning of the sleep state prediction model 311, which was performed by the Leave-one-out method using sleep stages assessed by polysomnography as teaching data.

In the above context, "simultaneously during polysomnography" means that acquisition of bio-vibration signals and polysomnography are started and finished at the same time. Medically established polysomnography records brain waves (electroencephalogram or EEG), electrooculogram (EOG), electromyogram (EMG), electrocardiogram (ECG), and oronasal air flows. At the end of recording, the recorded data are divided at an interval of 30 seconds into epochs, and a sleep stage is assessed as described above by an expert in accordance with internationally established sleep stage scoring criteria. The machine learning of the sleep state prediction model 311 pertinent to the embodiments is performed upon the obtained phase coherence ($\lambda$), body movement information (BM), heart rate (HR), and respiratory rate (RR) from the bio-vibration signals (electromyogram [EMG], electrocardiogram [ECG], and oronasal air flows) of the recorded data using the said sleep stage as teaching data.

The following explanation is related to a case in which a sleep state was predicted using phase coherence ($\lambda$), heart rate (HR), respiratory rate (RR), and body movement information (BM) as input data in the above sleep state prediction model 311.

1-1 Prediction using $\lambda$, HR, RR, and BM obtained from BCG as input data

To predict a sleep state, phase coherence ($\lambda$), heart rate (HR), respiratory rate (RR), and body movement information (BM) obtained from bio-vibration signals (ballistocardiogram or BCG) are used as input data in the sleep state prediction model 311 established by the above machine learning.

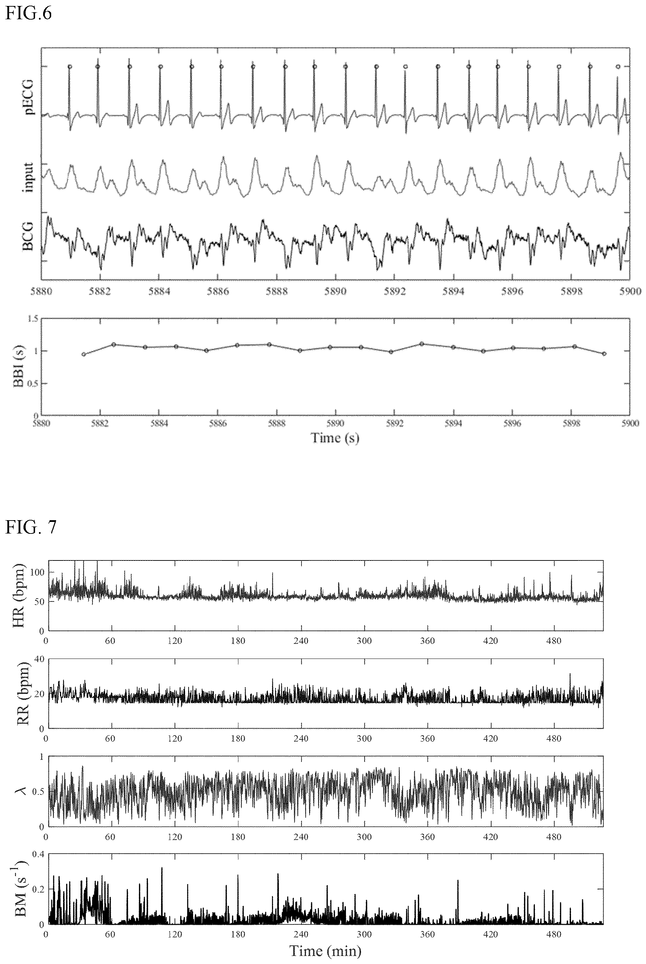

FIG. 7 illustrates signals representative of 10-second moving averages of the heart rate (HR), respiratory rate (RR), phase coherence ($\lambda$), and body movement (BM) obtained from bio-vibration signals (ballistocardiogram or BCG), which were acquired with a vibration sensor.

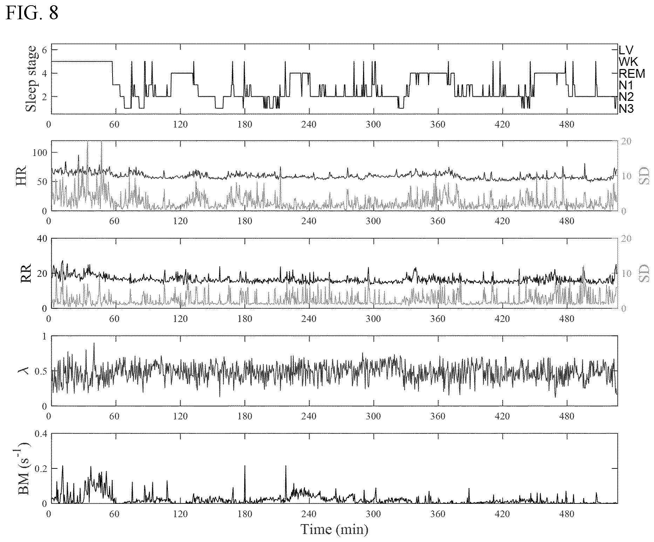

FIG. 8 illustrates input data for prediction of a sleep state using the sleep state prediction model 311 and sleep stages assessed by polysomnography (PSG). All the input data in the sleep state prediction model 311 were prepared by resampling information obtained from the bio-vibration signals (BCG) in FIG. 7 at an interval of 30 seconds and used for prediction of a sleep state.

The top chart in FIG. 8 illustrates sleep stages assessed by polysomnography (PSG), which serve ground truth data for evaluating a prediction result presented by the sleep state prediction model 311 through machine learning.

The second top chart illustrates heart rates (HRs) based on the bio-vibration signals (BCG) acquired with a piezoelectric sensor. For extraction of a heartbeat component, BCG signals were passed through a band-pass filter (BPF) with a pass-band of 5 Hz to 15 Hz for absolutization followed by smoothing with a time constant of 0.2 seconds and passing through a high-pass filter with the cutoff frequency of 0.3 Hz to eliminate drifts at low frequencies, and the subsequently obtained signals were used to derive predicted ECG signals, in which peaks were then counted to obtain the heart rate (HR).

The following explanation is related to a method to obtain predicted ECG signals.

Firstly, ECG signals measured with an electrocardiograph are acquired, and simultaneous bio-vibration signals (BCG) are acquired with a piezoelectric sensor. A prediction model is established by machine learning in which the ECG signals are used as teaching data, and model input signals obtained by specified processing of the bio-vibration signals (BCG) (bio-vibration signals [BCG], differentiated bio-vibration signals [BCG], heartbeat-derived vibration signals extracted from the bio-vibration signals [BCG], differentiated signals of the above heartbeat-derived vibration signals, and other signals) are input.

The bio-vibration signals (BCG) acquired with the vibration sensor (the third top chart in FIG. 6) were passed through a band-pass filter (BPF) with a pass-band of 5 Hz to 15 Hz for absolutization followed by smoothing with a time constant of 0.2 seconds and passing through a high-pass filter with the cutoff frequency of 0.3 Hz to eliminate drifts at low frequencies, and the subsequently obtained signals (the second top chart in FIG. 6) were input in an ECG prediction model to obtain predicted ECG (the top chart in FIG. 6), in which peaks were then counted to obtain the heartbeat interval (the bottom chart in FIG. 6).

The third top chart in FIG. 8 illustrates respiratory rates (RRs) based on the bio-vibration signals (BCG) acquired with the piezoelectric sensor.

The second bottom chart illustrates phase coherence ($\lambda$) calculated based on a difference in instantaneous phase between a variation in heartbeat interval and a breathing pattern. The bottom chart illustrates body movement information (BM) obtained from the bio-vibration signals (BCG).

All input data sets are prepared by sampling data at an interval of 30 seconds to match a time interval of the teaching data.

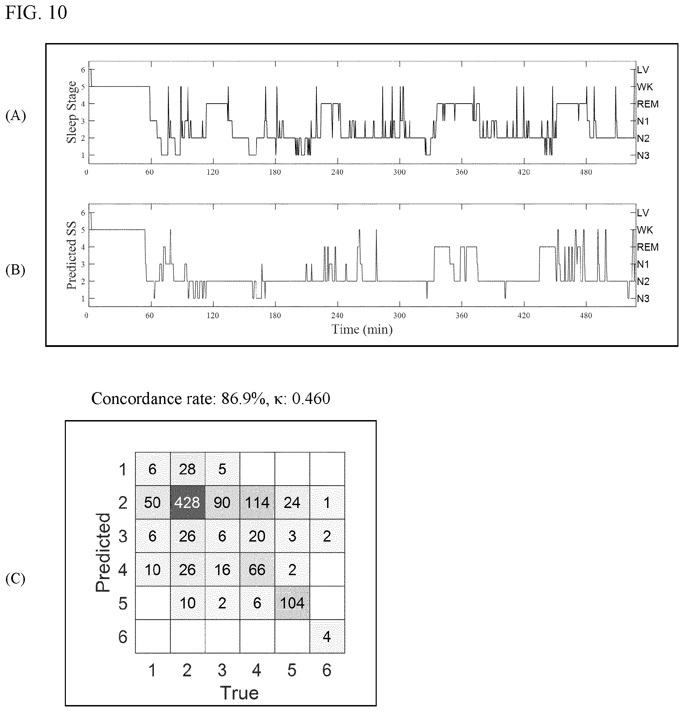

FIG. 10(A) illustrates measured sleep states. FIG. 10(B) illustrates prediction results obtained in response to input of phase coherence ($\lambda$), heart rate (HR), respiratory rate (RR), and body movement information (BM) obtained from the bio-vibration signals (BCG) in the sleep state prediction model 311, in which learning was performed according to the above BiLSTM neural network. FIG. 10(C) illustrates a confusion matrix between the prediction results and true values (measured values). As presented in FIG. 10(C), a concordance rate is 86.9% with a Cohen's Kappa coefficient of 0.460, indicating moderate agreement. As described above, the model in which learning had been performed according to BiLSTM neural network achieved favorable sleep state prediction.

Accuracy in prediction was evaluated with linear weighted concordance and the Cohen's Kappa coefficient. In this connection, the linear weighted concordance is determined by taking account of not only the absolute concordances but also the relative concordances. In the concerned EXAMPLE, linear weighting, prediction at the kth stage, a prediction result at the ith stage, and the true value at the jth stage are applied, leading to a weighting factor of $1 - |i - j|/(k - 1)$. More specifically, for the 6-stage sleep state (LV, WK, REM, N1, N2, N3), when a prediction result (predicted sleep state) and a true value (measured sleep state) agree ($i = j$), the weighing factor is 1; when these differ by 1 stage ($|i - j| = 1$), it is 4/5 (0.8); when these differ by 2 stages ($|i - j| = 2$), it is 3/5; when these differ by 3 stages ($|i - j| = 3$), it is 2/5; when these differ by 4 stages ($|i - j| = 4$), it is 1/5; and when these differ by 5 stages ($|i - j| = 5$), it is 0.

In addition, the Cohen's Kappa coefficient $\kappa$ represents an indicator for evaluating concordance between the prediction result (predicted sleep state) and the true value (measured sleep state) and is calculated according to $\kappa = (Po - Pe)/(1 - Pe)$ (where, Po = observed weighted concordance rate; Pe = accidental concordance rate). Criteria on a basis of the Cohen's Kappa coefficient $\kappa$ are as follows:

$\kappa$ <0: Poor agreement (poor)
$0< \kappa \leq 0.2$: Slight agreement (slight)
$0.2< \kappa \leq 0.4$: Fair agreement (fair)
$0.4< \kappa \leq 0.6$: Moderate agreement (moderate)
$0.6< \kappa \leq 0.8$: Substantial agreement (substantial)
$0.8< \kappa$: Perfect agreement (perfect)

1-2 Prediction using HR obtained from ECG as input data

Next, the sleep state was predicted using the following data sets as input data: phase coherence ($\lambda$) calculated from a difference in instantaneous phase between a variation in heartbeat interval based on an electrocardiogram (ECG) and a breathing pattern determined from respiratory rate (RR) based on bio-vibration signals (BCG) acquired with the piezoelectric sensor; heart rates (HRs) based on the ECG; respiratory rate (RR) based on BCG; and body movement (BM).

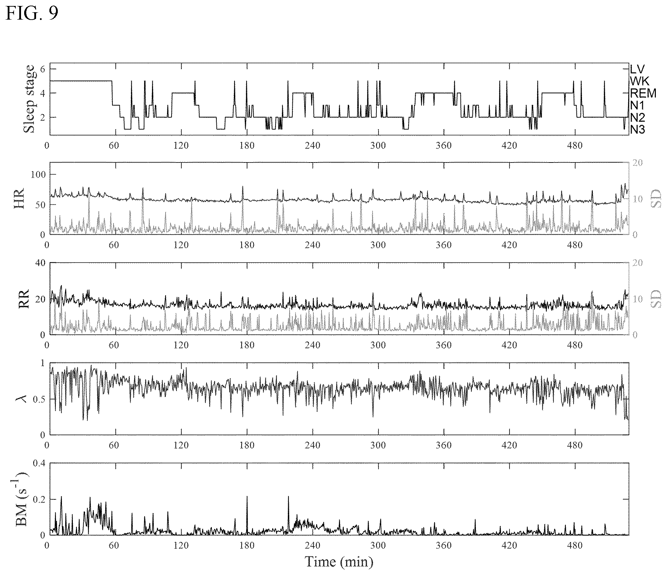

FIG. 9 illustrates sleep stages assessed based on the input data for predicting a sleep state with the sleep state prediction model 311 and by polysomnography (PSG).

The top chart in FIG. 9 illustrates sleep stages assessed by polysomnography (PSG), which serve ground truth data for evaluating a prediction result presented by the sleep state prediction model 311 through machine learning.

The second top chart in FIG. 9 illustrates heart rates (HRs) based on the electrocardiogram (ECG).

The third top chart in FIG. 9 illustrates respiratory rates (RRs) based on the bio-vibration signals (BCG) acquired with the piezoelectric sensor.

The second bottom chart illustrates phase coherence ($\lambda$) calculated from a difference in instantaneous phase between the variation in heartbeat interval based on the electrocardiogram (ECG) and the breathing pattern extracted from the bio-vibration signals (BCG) acquired with the piezoelectric sensor.

The bottom chart illustrates body movement (BM) signals obtained from the bio-vibration signals (BCG).

All input data sets are prepared by sampling data at an interval of 30 seconds to match a time interval of the teaching data.

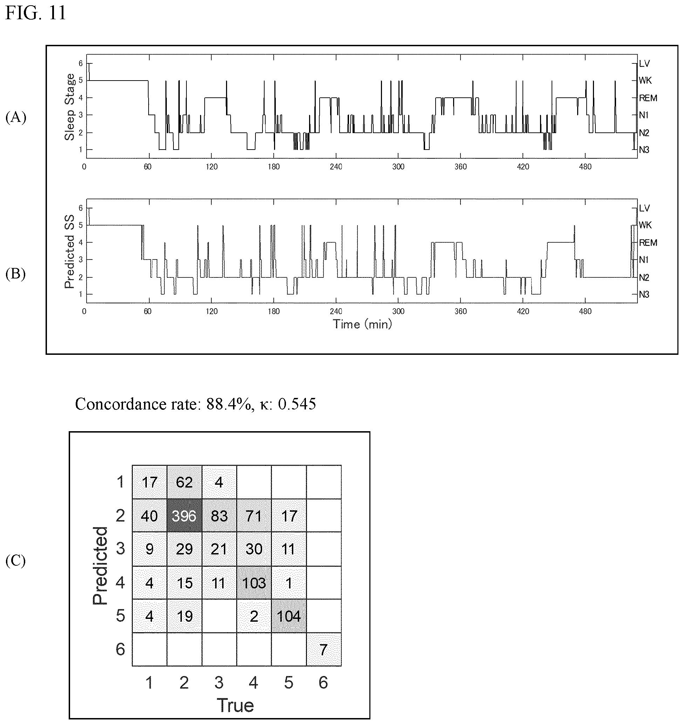

FIG. 11(A) illustrates measured sleep states. FIG. 11(B) illustrates prediction results obtained in response to input of phase coherence, respiratory rate, and heart rate based on ECG as well as body movement (BM) based on the BCG in the sleep state prediction model 311, in which learning was performed according to the above BiLSTM neural network. FIG. 11(C) illustrates a confusion matrix between the prediction results and true values (measured values). As presented in FIG. 11(C), a concordance rate is 88.4% with a Cohen's Kappa coefficient of 0.545, indicating moderate agreement. As described above, the model in which learning had been performed according to BiLSTM neural network achieved substantial sleep state prediction.

EXAMPLE 2 Prediction using $\lambda$, RR, and BM based on BCG as input data

Bio-vibration signals were acquired from each subject simultaneously during polysomnography, and from the said signals, phase coherence ($\lambda$), body movement information (BM), and respiratory rate (RR) were obtained and subjected to machine learning of the sleep state prediction model 311, which was performed by the Leave-one-out method using sleep stages assessed by polysomnography as teaching data.

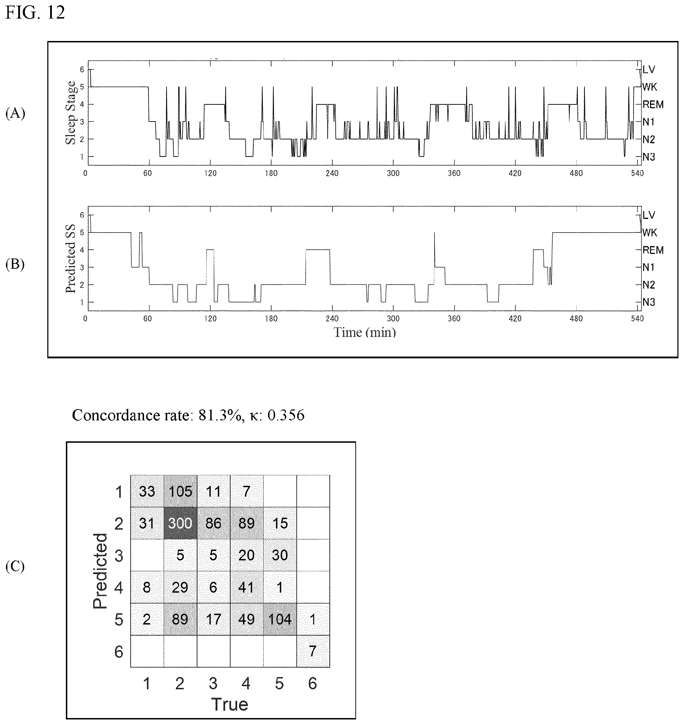

FIG. 12(A) illustrates sleep stages (measured values) assessed by polysomnography. FIG. 12(B) illustrates prediction results obtained in response to input of phase coherence ($\lambda$), respiratory rate (RR), and body movement information (BM) based on the bio-vibration signals (BCG) in the sleep state prediction model 311, in which learning was performed according to the above BiLSTM neural network. FIG. 12(C) illustrates a confusion matrix between the prediction results and true values (measured values). As presented in FIG. 12(C), a concordance rate is 81.3% with a Cohen's Kappa coefficient of 0.356, indicating fair agreement. As described above, the model in which learning had been performed according to BiLSTM neural network achieved substantial sleep state prediction even when 3 data sets were used as input data.

EXAMPLE 3 Prediction using λ, HR, and BM based on BCG as input data Bio-vibration signals were acquired from each subject simultaneously during polysomnography, and from the said signals, phase coherence (λ), body movement information (BM), and heart rate (HR) were obtained and subjected to machine learning of the sleep state prediction model 311, which was performed by the Leave-one-out method using sleep stages assessed by polysomnography as teaching data.

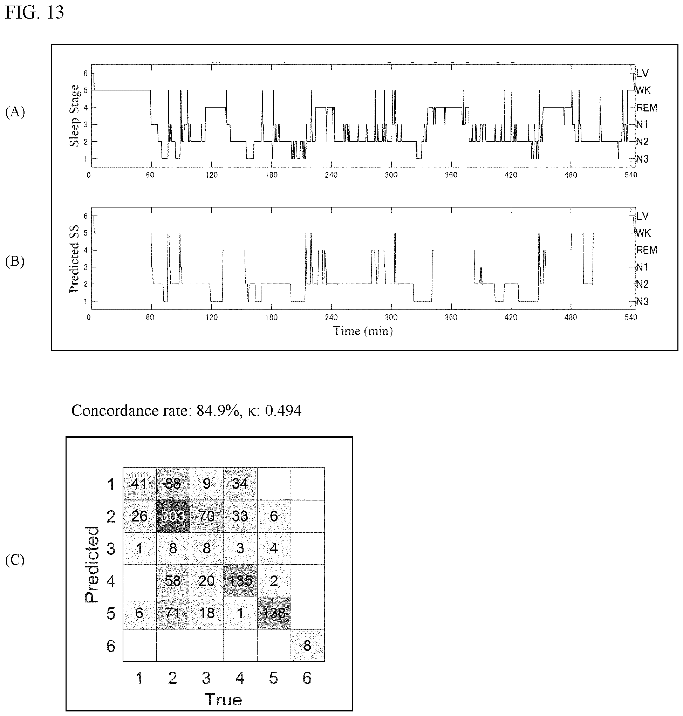

FIG. 13(A) illustrates sleep stages (measured values) assessed by polysomnography. FIG. 13(B) illustrates prediction results obtained in response to input of phase coherence (λ), heart rate (HR), and body movement information (BM) based on the bio-vibration signals (BCG) in the sleep state prediction model 311, in which learning was performed according to the above BiLSTM neural network. FIG. 13(C) illustrates a confusion matrix between the prediction results and true values (measured values). As presented in FIG. 13(C), a concordance rate is 84.9% with a Cohen's Kappa coefficient of 0.494, indicating moderate agreement. As described above, the model in which learning had been performed according to BiLSTM neural network achieved substantial sleep state prediction even when 3 data sets were used as input data.

EXAMPLE 4 Prediction using λ and BM based on BCG as input data

Bio-vibration signals were acquired from each subject simultaneously during polysomnography, and from the said signals, phase coherence (λ) and body movement information (BM) were obtained and subjected to machine learning of the sleep state prediction model 311, which was performed by the Leave-one-out method using sleep stages assessed by polysomnography as teaching data.

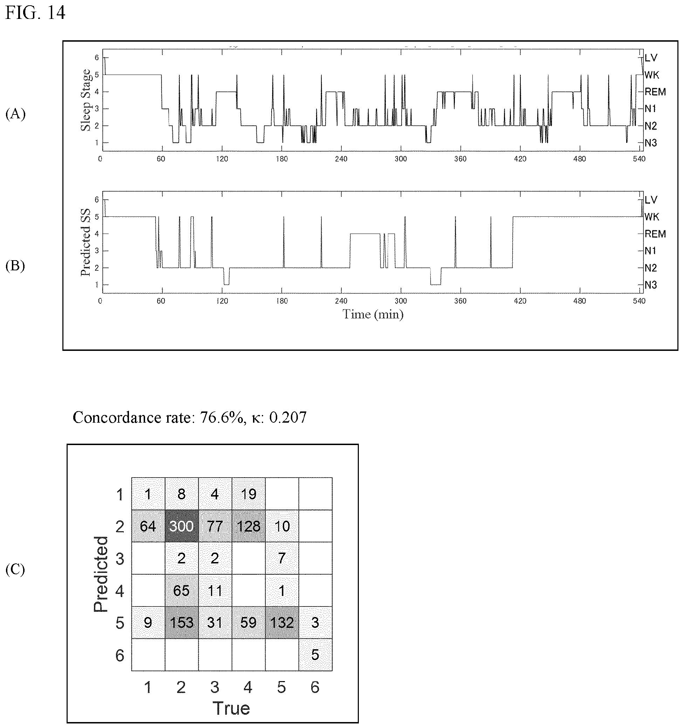

FIG. 14(A) illustrates sleep stages (measured values) assessed by polysomnography. FIG. 14(B) illustrates prediction results obtained in response to input of phase coherence (λ) and body movement information (BM) based on the bio-vibration signals (BCG) in the sleep state prediction model 311, in which learning was performed according to the above BiLSTM neural network. FIG. 14(C) illustrates a confusion matrix between the prediction results and true values (measured values). As presented in FIG. 14(C), a concordance rate is 76.6% with a Cohen's Kappa coefficient of 0.207, indicating fair agreement. As described above, the model in which learning had been performed according to BiLSTM neural network achieved substantial sleep state prediction even when 2 data sets were used as input data.

EXAMPLE 5 Determination of heart rate (HR) by CWT processing on bio-vibration signals (BCG)

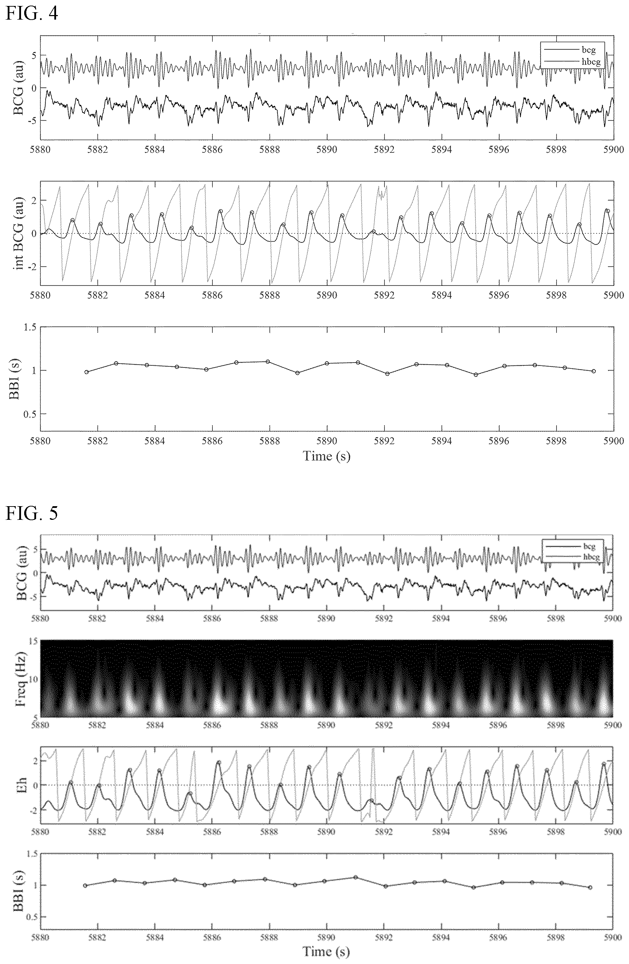

The bio-vibration signals (BCG) were subjected to continuous wavelet transform (CWT) to derive frequency components (the second top chart in FIG. 5), and of these, a 5-15 Hz component was integrated to obtain signals (the third top chart in FIG. 5), from which, heartbeat intervals (the bottom chart in FIG. 5) were determined.

In a subsequent process, sleep states can be predicted as done in EXAMPLES 1 and 3.

EXAMPLE 6 Determination of heart rate (HR) by passing bio-vibration signals (BCG) through band-pass filter

The bio-vibration signals (BCG) were passed through a band-pass filter with a pass-band of 5 Hz to 15 Hz followed by full-wave rectification integration at a time constant of 0.1 seconds and passing through a high-pass filter with the cutoff frequency of 0.3 Hz to determine heartbeat intervals (the bottom chart in FIG. 4). In a subsequent process, sleep states can be predicted as done in EXAMPLES 1 and 3.

In these examples, use of a standard deviation of either of heart rate (HR) or respiratory rate (RR) or both can improve the accuracy of sleep state prediction.

In addition, phase coherence, heart rate, and respiratory rate based on pulse waves and phonocardiogram may be also used to obtain input data in the sleep state prediction model 311 for prediction.

Even after machine learning has been performed, the sleep state prediction model 311, while predicting sleep states, can undergo further machine learning using sleep stages assessed by new polysomnography as teaching data as well as at least 2 sets of data on phase coherence, body movement, heart rate, and respiratory rate obtained from simultaneous bio-vibration signals as input data to improve prediction performance.

Although the subjects were humans in these examples, not only humans but also a wide range of animals such as dogs, cats, and other companion animals may be eligible.

[Reference Signs List]

1 Sleep state measuring system
2 Information acquisition unit
3 Information processing unit
4 Memory unit
5 Operation unit
6 Output unit
22 Phase coherence acquisition device
23 Body movement information acquisition device
24 Heartbeat information acquisition device
25 Respiratory information acquisition device
26 Sleep state information acquisition device
31 Sleep state prediction device
32 Phase coherence calculation device
33 Body movement information calculation device
34 Heartbeat information calculation device
35 Respiratory information calculation device
36 Sleep state prediction model learning device
41 Primary database
311 Sleep state prediction model

**Claims**

1. A sleep state prediction system for predicting a sleep state of an animal from calculation results presented by a sleep state prediction model, the system comprising:

   A phase coherence acquisition device that acquires phase coherence calculated based on a difference in instantaneous phase between a variation in heartbeat interval and a breathing pattern simultaneously obtained from the animal during sleep;
   A body movement information acquisition device that acquires body movement information from the animal simultaneously with the variation in heartbeat interval, a heartbeat information acquisition device that acquires heartbeat information from the animal simultaneously with the variation in heartbeat interval, or a respiratory information acquisition device that acquires respiratory information from the animal simultaneously with the variation in heartbeat interval ; and
   A sleep state prediction device that has a sleep state prediction model built-in, which is established through machine learning using sleep stages assessed by polysomnography on the animal as teaching data and at least 2 sets of data on 4 parameters of phase coherence, body movement, heartbeat, and respiration determined from bio-vibration signals acquired simultaneously during the polysomnography as input data.
   The sleep state prediction device inputs in the sleep state prediction model the same type of data as that of the input data, which are chosen from data on the following parameters, and the said sleep state prediction model presents calculation results for the prediction: a variation in heartbeat interval the phase coherence acquisition device obtained from the heartbeat information acquisition device; phase coherence calculated from the breathing pattern obtained from the respiratory information acquisition device; body movement obtained from the body movement information acquisition device; heartbeat obtained from the heartbeat information acquisition device; and respiration obtained from the respiratory information acquisition device.

2. The sleep state prediction system described in Claim 1, comprising the phase coherence acquisition device that obtains phase coherence calculated based on a difference in instantaneous phase between a variation in heartbeat interval acquired with the heartbeat information acquisition device and a breathing pattern acquired with the respiratory information acquisition device.

3. The sleep state prediction system described in either Claim 1 or Claim 2, comprising the heartbeat information acquisition device that acquires heart rate as the heartbeat information; and the respiratory information acquisition device that acquires respiratory rate as the respiratory information.

4. The sleep state prediction system described in any of Claims 1 to 3, comprising the heartbeat information acquisition device that acquires the variation in heartbeat interval and the heart rate calculated from predicted ECG signals based on bio-vibration signals, which have been used as input data.

5. The sleep state prediction system described in any of Claims 1 to 3, comprising the heartbeat information acquisition device that acquires the variation in heartbeat interval and the heart rate calculated from signals obtained by integration of a power of the frequency component ranging from 5 Hz to 15 Hz, which is a part of the frequency components derived from bio-vibration signals by continuous wavelet transform (CWT).

6. The sleep state prediction system described in any of Claims 1 to 3, comprising the heartbeat information acquisition device that acquires the variation in heartbeat interval and the heart rate calculated from signals obtained by passing bio-vibration signals through a band-pass filter with a pass-band of 5 Hz to 15 Hz.

7. The sleep state prediction system described in any of Claims 1 to 3, comprising the heartbeat information acquisition device that acquires the variation in heartbeat interval and the heart rate calculated from pulse waves or signals in a phonocardiogram.

8. The sleep state prediction system described in any of Claims 1 to 7, comprising the respiratory information acquisition device that acquires the breathing pattern and the respiratory rate calculated from signals obtained by passing bio-vibration signals through a low-pass filter with the upper frequency of 0.5 Hz.

9. The sleep state prediction system described in any of Claims 1 to 8, comprising the body movement information acquisition device that acquires the number of body movements (the number of peaks exceeding a predetermined threshold among bio-vibration signals), the number of body movements in the predetermined period of time, or the integration value of body movement signals in the predetermined period of time as the body movement information.

10. The sleep state prediction system described in any of Claims 1 to 9, comprising the heartbeat information acquisition device that acquires the heartbeat information additionally including data on standard deviation of heart rate; and the respiratory information acquisition device that acquires the respiratory information additionally including data on standard deviation of respiratory rate.

FIG.1

Information acquisition unit

22 — Phase coherence acquisition device

23 — Body movement information acquisition device

24 — Heartbeat information acquisition device

25 — Respiratory information acquisition device

26 — Sleep state information acquisition device

Information processing unit — 3

Sleep state prediction device — 31

Sleep state prediction model — 311

Phase coherence calculation device — 32

Body movement information calculation device — 33

Heartbeat information calculation device — 34

Respiratory information calculation device — 35

Sleep state prediction model learning device — 36

Memory unit — 4

Primary database — 41

Operation unit — 5

Output unit — 6

FIG.2

```
           ┌──────────────────────────────┐
           │ Phase coherence              │
           │ Body movement information    │
           │ Heartbeat information        │
           │ Respiratory information      │
           └──────────────────────────────┘
   2                    │                        311
     ┌───────────┐      │      ┌───────────┐           ┌───────────┐
     │Information│      ▼      │Sleep state│           │           │
     │acquisition│ ──Input──▶  │prediction │ ─Output─▶ │Sleep state│
     │   unit    │             │  model    │           │           │
     └───────────┘             └───────────┘           └───────────┘
```

(A) Prediction mode

```
              ┌────────────┐   ┌──────────────────────────────┐
              │            │   │ Phase coherence              │
              │Sleep state │   │ Body movement information    │
              │            │   │ Heartbeat information        │
              │            │   │ Respiratory information      │
              └────────────┘   └──────────────────────────────┘
  41                  │                    │                     311
     ┌───────────┐    └──────────┬─────────┘       ┌───────────┐
     │  Primary  │               ▼                 │Sleep state│
     │ database  │ ──────────Input──────────▶       │prediction │
     └───────────┘                                 │  model    │
                                                   └───────────┘
```

(B) Learning mode

FIG.3

**(A)**

Start

S1 — Acquisition of phase coherence

S2 — Data input in sleep state prediction model

S3 — Calculation by sleep state prediction model

S4 — Prediction of sleep state

End

**(B)**

Start

S11 — Acquisition of bio-vibration signals

S12 — Calculation of body movement, heartbeat, and respiratory information

S13 — Calculation of phase coherenc

S14 — Data normalization

S15 — Data input in sleep state prediction model

S16 — Calculation by sleep state prediction model

S17 — Prediction of sleep state

End

FIG. 4

FIG. 5

FIG.6

FIG. 7

FIG. 8

FIG. 9

FIG. 10

(A)

(B)

Concordance rate: 86.9%, κ: 0.460

(C)

FIG. 11

(A)

(B)

Concordance rate: 88.4%, κ: 0.545

(C)

FIG. 12

(A)

(B)

(C)

Concordance rate: 81.3%, κ: 0.356

FIG. 13

(A)

(B)

Concordance rate: 84.9%, κ: 0.494

(C)

FIG. 14

(A)

(B)

Concordance rate: 76.6%, κ: 0.207

(C)

| INTERNATIONAL SEARCH REPORT | International application No. |
|---|---|
| | PCT/JP2021/025053 |

**A. CLASSIFICATION OF SUBJECT MATTER**

A61B 5/16(2006.01)i
FI: A61B5/16 130

According to International Patent Classification (IPC) or to both national classification and IPC

**B. FIELDS SEARCHED**

Minimum documentation searched (classification system followed by classification symbols)
A61B5/00-5/398

Documentation searched other than minimum documentation to the extent that such documents are included in the fields searched

| Published examined utility model applications of Japan | 1922-1996 |
|---|---|
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | US 2015/0190086 A1 (VITAL CONNECT, INC.) 09 July 2015 (2015-07-09) paragraphs [0015]-[0030] | 1-10 |
| Y | JP 2017-64338 A (HEALTH SENSING CO LTD) 06 April 2017 (2017-04-06) paragraphs [0025]-[0037], [0048], [0050], [0060]-[0061] | 1-10 |
| Y | JP 2004-89267 A (RITSUMEIKAN) 25 March 2004 (2004-03-25) paragraph [0011] | 1-10 |
| Y | JP 2020-75136 A (HEALTH SENSING CO LTD) 21 May 2020 (2020-05-21) paragraphs [0022]-[0025], [0029]-[0030] | 5, 7 |
| A | JP 5-95934 A (MATSUSHITA ELECTRIC IND CO LTD) 20 April 1993 (1993-04-20) entire text, all drawings | 1-10 |
| A | WO 2017/134681 A2 (PARCHANI, Gaurav) 10 August 2017 (2017-08-10) entire text, all drawings | 1-10 |

☐ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 27 August 2021 (27.08.2021) | 07 September 2021 (07.09.2021) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**
Information on patent family members

International application No.

PCT/JP2021/025053

| Patent Documents referred in the Report | Publication Date | Patent Family | Publication Date |
|---|---|---|---|
| US 2015/0190086 A1 | 09 Jul. 2015 | WO 2015/103558 A1 | |
| JP 2017-64338 A | 06 Apr. 2017 | JP 2019-134953 A | |
| JP 2004-89267 A | 25 Mar. 2004 | (Family: none) | |
| JP 2020-75136 A | 21 May 2020 | (Family: none) | |
| JP 5-95934 A | 20 Apr. 1993 | (Family: none) | |
| WO 2017/134681 A2 | 10 Aug. 2017 | (Family: none) | |

Form PCT/ISA/210 (patent family annex) (January 2015)

**EP 4 327 743 A1**

## REFERENCES CITED IN THE DESCRIPTION

### Patent documents cited in the description

- JP 6358768 B